(19) Europäisches Patentamt
European Patent Office
Office européen des brevets

(11) Publication number: **0 153 828**
**B1**

(12) **EUROPEAN PATENT SPECIFICATION**

(45) Date of publication of patent specification: **27.12.90**

(21) Application number: **85300832.4**

(22) Date of filing: **08.02.85**

(51) Int. Cl.[5]: **C 07 D 471/04,**
C 07 D 215/56,
C 07 D 401/04,
A 61 K 31/495, A 61 K 31/55,
A 61 K 31/535, A 61 K 31/54,
A 61 K 31/47 // (C07D471/04,
221:00)

(54) **1-cyclopropyl-1, 4-dihydro-6-fluoro-4-oxo-1, 8-naphthyridine-3-carboxylic acids; their derivatives and a process for preparing the compounds.**

(30) Priority: **17.02.84 US 581410**
**23.01.85 US 692819**

(43) Date of publication of application:
**04.09.85 Bulletin 85/36**

(45) Publication of the grant of the patent:
**27.12.90 Bulletin 90/52**

(84) Designated Contracting States:
**AT BE CH DE FR GB IT LI LU NL SE**

(56) References cited:
**EP-A-0 009 425**
**EP-A-0 049 355**
**EP-A-0 126 355**
**US-A-4 398 029**

(73) Proprietor: **WARNER-LAMBERT COMPANY**
**201 Tabor Road**
**Morris Plains New Jersey 07950 (US)**

(72) Inventor: **Mich, Thomas F.**
**915 Sunset Road**
**Ann Arbor Michigan 48103 (US)**
Inventor: **Sanchez, Joseph P.**
**739 Meadowlake Road**
**Canton Michigan 48188 (US)**
Inventor: **Domagala, John M.**
**776 Georgetown**
**Canton Michigan 48188 (US)**
Inventor: **Trehen, Ashok K.**
**3710 Green Brier Blvd., Apt. 370C**
**Ann Arbor Michigan 48105 (US)**

(74) Representative: **Jones, Michael Raymond et al**
**HASELTINE LAKE & CO. Hazlitt House 28**
**Southampton Buildings Chancery Lane**
**London WC2A 1AT (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 153 828 B1

Courier Press, Leamington Spa, England.

## Description

Certain 7-heterocyclic substituted 1,8-naphthyridines are disclosed in Eur. J. Med. Chem. — Chimica Therapeutica, *29*, 27 (1977).

EP—0049355 discloses 7-amino-1-cyclopropyl-4-oxo-1,4-dihydro-naphthyridine-3-carboxylic acids useful as antibacterial agents.

The above references teach that these compounds possess antibacterial activity.

The present invention relates to a compound of the formula I

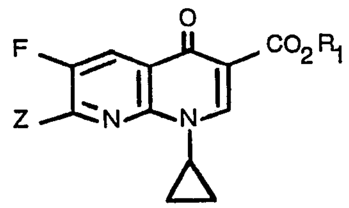

(I)

wherein Z is a leaving group or a group of the formula

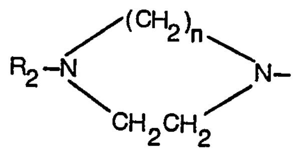

where n is 2—3 and $R^2$ is hydrogen, $C_1$—$C_3$ alkyl or acetyl,

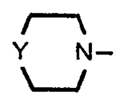

where Y is O or S, or

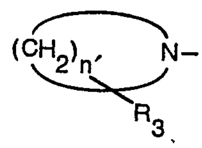

where n′ is 4—6 and $R^3$ is hydrogen or hydroxyl; $R^1$ is hydrogen or $C_1$—$C_3$ alkyl, or a pharmaceutically acceptable acid-addition or base salt thereof.

The present invention includes, as novel intermediates, 1-cyclopropyl-6,7-difluoro-1,4-dihydro-4-oxo-1,8-naphthyridine-3-carboxylic acid, and 1-cyclopropyl-6-fluoro-7-chloro-1,4-dihydro-4-oxo-1,8-naphthyridine-3-carboxylic acid as well as $C_1$—$C_3$ alkyl esters or salts thereof.

The invention also includes a pharmaceutical composition which comprises an antibacterially effective amount of a compound having structural formula I and the pharmaceutically acceptable acid-addition or base salts thereof in combination with a pharmaceutically acceptable carrier.

The compounds of the invention (other than those in which Z is a leaving group) may be used in a method of treating bacterial infections in a mammal which comprises administering an antibacterially effective amount of the above defined pharmaceutical composition to a mammal in need thereof. The compounds in which Z is a leaving group are useful intermediates.

The compounds of the invention having the structural formula I may be readily prepared by treating a corresponding compound having the structural formula II

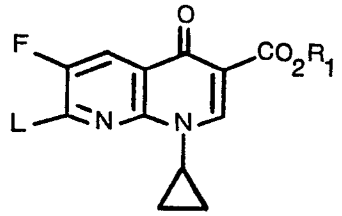

(II)

wherein $R_1$ is as defined above and L is a leaving group, preferably fluorine or chlorine, with an amine corresponding to the group Z.

If the group Z contains an alkylamine substituent, said substituent may, if desired, be protected by a group which renders it substantially inert to the reaction conditions. Thus, for example, protecting groups such as the following may be utilized:

carboxylic acyl groups such as formyl, acetyl, trifluoroacetyl;

alkoxycarbonyl, groups such as ethoxycarbonyl, t-butoxycarbonyl, β,β,β-trichlorethoxycarbonyl, β-iodoethoxycarbonyl;

aryloxycarbonyl groups such as benzyloxycarbonyl, *p*-methoxybenzyloxycarbonyl, phenoxycarbonyl;

silyl groups such as trimethylsilyl; and groups such as trityl, tetrahydropyranyl, vinyloxycarbonyl, *o*-nitrophenylsulfenyl, diphenylphosphinyl, *p*-toluenesulfonyl, and benzyl, may all be utilized.

The protecting group may be removed, after the reaction if desired, by procedures known to those skilled in the art. For example, the ethoxycarbonyl group may be removed by acid or base hydrolysis and the trityl group may be removed by hydrogenolysis.

The reaction between the compound of structural formula II and a suitably protected amine, if necessary, of group Z may be performed with or without a solvent, preferably at elevated temperature for a sufficient time so that the reaction is substantially complete. The reaction is preferably carried out in the presence of an acid acceptor such as an alkali metal or alkaline earth metal carbonate or bicarbonate, a tertiary amine such as triethylamine, pyridine, or picoline. Alternatively an excess of the amine of the group Z may be utilized as the acid acceptor.

Convenient solvents for this reaction are non-reactive solvents such as acetonitrile, tetrahydrofuran, ethanol, chloroform, dimethylsulfoxide, dimethylformamide, pyridine, picoline or water. Solvent mixtures may also be utilized.

Convenient reaction temperatures are in the range of from 20° to 150°C; higher temperatures usually require shorter reaction times.

The removal of the protecting group may be accomplished either before or after isolating the product.

Alternatively, the compound of formula I wherein $R_1$ is hydrogen and Z is piperazine may be prepared by removal of its precursor carboethoxypiperazine derivative and/or ester thereof. The piperazine may then be alkylated by known means to form the $C_1$—$C_3$ alkyl piperazine derivatives of formula I.

The above compound, namely 1-cyclopropyl-6-fluoro-1,4-dihydro-4-oxo-7-(1-piperazinyl)-1,8-naphthyridine-3-carboxylic acid, its 4-$C_1$—$C_3$ alkylpiperazinyl, or its 4-carboethoxypiperazinyl derivative and/or esters thereof are also useful as intermediates to prepare a compound of formula II wherein X is N and L is fluorine or chlorine. The piperazine groups may be cleaved and displaced by a hydroxyl group by treating with a mixture of nitric and sulfuric acids, which hydroxyl compound is further displaced by group L, fluorine, or chlorine. For example, treatment of the hydroxyl compound with phosphorus-oxychloride under known conditions affords the chloro compound of formula II.

The starting material 1-cyclopropyl-6-fluoro-1,4-dihydro-4-oxo-7-[1-(4-carboethoxy)piperazinyl]1,8-naphthyridine-3-carboxylic acid and its ethyl ester may be prepared as described in the Preparative Examples.

The amines corresponding to group Z are known and either commercially available or capable of being prepared by methods known in the art.

The compounds of the invention (other than those in which Z is a leaving group) display antibacterial activity when tested by the microtitration dilution method as described in Heifetz, et al, Antimicr. Agents & Chemoth., *6*, 124 (1974).

By use of the above referenced method, the followed minimum inhibitory concentration values (MICs in µg/ml) were obtained for representative compounds of the invention.

IN VITRO ANTIBACTERIAL ACTIVITY
Minimal Inhibitory Concentration
MIC (μg/ml)

| Organisms | Compound Ex. 1 | Compound Ex. 2 |
|---|---|---|
| Enterobacter cloacae MA 2646 | 0.1 | ≤0.1 |
| Escherichia coli Vogel | ≤0.1 | ≤0.1 |
| Klebsiella pneumoniae MGH—2 | 0.1 | ≤0.1 |
| Proteus rettgeri M 1771 | 0.2 | 0.4 |
| Pseudomonas aeruginosa UI—18 | 0.2 | 0.4 |
| Staphylococcus aureus H 228 | 1.6 | 0.4 |
| Staphylococcus aureus UC—76 | 0.4 | 0.1 |
| Streptococcus faecalis MGH—2 | 1.6 | 0.8 |
| Streptococcus pneumoniae SV—1 | 1.6 | 6.3 |
| Streptococcus pyogenes C—203 | 1.6 | 3.1 |

The compounds of the invention are capable of forming both pharmaceutically acceptable acid addition and/or base salts. Base salts are formed with metals or amines, such as alkali and alkaline earth metals or organic amines. Examples of metals used as cations are sodium, potassium, magnesium or calcium. Examples of suitable amines are N,N'-dibenzylethylenediamine, chloroprocaine, choline, diethanolamine, ethylenediamine, N-methylglucamine, and procaine.

Pharmaceutically acceptable acid addition salts are formed with organic and inorganic acids.

Examples of suitable acids for salt formation are hydrochloric, sulfuric, phosphoric, acetic, citric, oxalic, malonic, salicylic, malic, gluconic, fumaric, succinic, ascorbic, maleic or methanesulfonic acid. The salts are prepared by contacting the free base form with a sufficient amount of the desired acid to produce either a mono or di, etc. salt in the conventional manner. The free base forms may be regenerated by treating the salt form with a base. For example, dilute solutions of aqueous base may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention. Use of excess base where $R_1$ is hydrogen gives the corresponding basic salt.

The compounds of the invention can exist in unsolvated as well as solvated forms, including hydrated forms. In general, the solvated forms, including hydrated forms are equivalent to the unsolvated forms for purposes of the invention.

Certain compounds of the invention may exist in optically active forms. The pure D isomer, pure L isomer as well as mixtures thereof; including the racemic mixtures, are contemplated by the invention. Additional assymmetric carbon atoms may be present in a substituent such as an alkyl group. All such isomers as well as mixtures thereof are intended to be included in the invention.

A preferred class of compounds of the present invention are those of formula I where Z is 1-piperazinyl or 4-$C_1$—$C_3$ alkyl-1-piperazinyl. Particularly preferred are those compounds of formula I wherein Z is 1-piperazinyl or 4-methyl-1-piperazinyl and their pharmaceutically acceptable salts.

The compounds of the invention can be prepared and administered in a wide variety of oral and parenteral dosage forms. It will be obvious to those skilled in the art that the following dosage forms may comprise as the active component, either a compound of formula I or a corresponding pharmaceutically acceptable salt of a compound of formula I.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders, or tablets disintegrating agents; it can also be an encapsulating material. In powders, the carrier is a finely divided solid which is in admixture with the finely divided active compound. In the tablet the active

compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax or cocoa butter. The term "preparation" is intended to include the formulation of the active compound with encapsulating material as carrier providing capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions suspensions and emulsions. As an example may be mentioned water or water-propylene glycol solutions for parenteral injection. Such solutions are prepared so as to be acceptable to biological systems (isotonicity, pH). Liquid preparations can also be formulated in solution in aqueous polyethylene glycol solution. Aqueous solutions suitable for oral use can be prepared by dissolving the active component in water and adding suitable colorants, flavors, stabilizing, and thickening agents as desired. Aqueous suspension suitable for oral use can be made by dispersing the finely divided active component in water with viscous material, i.e., natural or synthetic gums, resins, methyl cellulose, sodium carboxymethyl cellulose, and other well-known suspending agents.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules, and powders in vials or ampoules. The unit dosage form can also be a capsule, cachet, or tablet itself or it can be the appropriate number of any of these packaged forms.

The quantity of active compound in a unit dose of preparation may be varied or adjusted from 1 mg to 100 mg according to the particular application and the potency of the active ingredient.

In therapeutic use as agents for treating bacterial infections the compounds utilized in the pharmaceutical method of this invention are administered at the initial dosage of about 3 mg to about 40 mg per kilogram daily. A daily dose range of about 6 mg to about 14 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following nonlimiting examples illustrate the inventors' preferred methods for preparing the compounds of the invention. (Those Examples preceded by the word "Reference" involve the preparation of compounds not in accordance with the invention, but useful as reaction protocols.)

### Example 1

Route A

1-Cyclopropyl-6-fluoro-1,4-dihydro-4-oxo-7-(1-piperazinyl)-1,8-naphthyridine-3-carboxylic acid.

A suspension of 0.7 g (1.6 mmole) of ethyl 1 - cyclopropyl - 6 - fluoro - 1,4 - dihydro - 4 - oxo - 7 - [4 - (ethoxycarbonyl) - 1 - piperazinyl] - 1,8 - naphthyridine - 3 - carboxylate (see Example H), 6 ml of 10% aqueous sodium hydroxide and 2 ml of ethanol was refluxed for three hours. The reaction was filtered through a fiber glass pad to clarify and acidified to pH 1.5 with 6.0 M hydrochloric acid and lyophilized. The residue was dissolved in 10 ml of ammonium hydroxide and the solution concentrated in vacuo. The precipitate which formed was removed by filtration, washed with aqueous ethanol, ether and dried in vacuo to give 0.04 g of the title compound, mp 274—276°C.

Route B

1-Cyclopropyl-6-fluoro-1,4-dihydro-4-oxo-7-(1-piperazinyl)-1,8-naphthyridine-3-carboxylic acid.

A solution of 10.2 g (25 mmole) of 1 - cyclopropyl - 6 - fluoro - 1,4 - dihydro - 4 - oxo - 7 - [4 - (ethoxycarbonyl) - 1 - piperazinyl] - 1,8 - naphthyridine - 3 - carboxylic acid (see Example J), 100 ml of 10% aqueous sodium hydroxide and 40 ml of ethanol was refluxed for three hours. The solution was concentrated to 125 ml and acidified to pH 7.3 with glacial acetic acid. The resulting precipitate was removed by filtration, washed with 50% aqueous ethanol, ether and dried in vacuo to give 7.2 g of the title compound, mp 274—276°C.

### Example 2

1-Cyclopropyl-6-fluoro-1,4-dihydro-7-(4-methyl-1-piperazinyl)-4-oxo-1,8-naphthyridine-3-carboxylic acid.

A suspension of 1.3 g (4.0 mmole) of 1 - cyclopropyl - 6 - fluoro - 1,4 - dihydro - 4 - oxo - 7 - (1 - piperazinyl) - 1,8 - naphthyridine - 3 - carboxylic acid, 13.3 ml of 37% formalin and 13.3 ml of 88% formic acid was refluxed for four hours. The resulting solution was evaporated in vacuo. The residue was suspended in aqueous ethanol, the resulting precipitate removed by filtration, washed with water and dried in vacuo to give 1.24 g of the title compound, mp 236—237°C.

Reference Example 3

1-Cyclopropyl-6,8-difluoro-1,4-dihydro-4-oxo-7-(1-piperazinyl)-3-quinolinecarboxylic acid

To 1.00 g (3.53 mmol) of 1 - cyclopropyl - 6,7,8 - trifluoro - 1,4 - dihydro - 4 - oxo - 3 - quinolinecarboxylic acid (see Example L) in 10.0 ml of acetonitrile and 0.54 g (3.53 mmol) of 1,8 - diazabicyclo[5.4.0]undec - 7 - ene, was added 1.70 g (19.7 mmol) of piperazine. The mixture was refluxed for one hour and then stirred overnight. It was concentrated, dissolved in ammonium hydroxide and filtered. The filtrate was then concentrated to one-half volume and filtered to give 0.67 g of the title compound, mp >270°C.

Example 4

In the same manner as Reference Example 3, the following compounds are prepared from 1 - cyclopropyl - 6 - fluoro - 7 - chloro - 1,4 - dihydro - 4 - oxo - 1,8 - naphthyridine - 3 - carboxylic acid (see Example K) and the desired amine or blocked amine:

1-cyclopropyl-1,4-dihydro-6-fluoro-4-oxo-7-(4-thiomorpholinyl)-1,8-naphthyridine-3-carboxylic acid, mp 288—291°C.

1-cyclopropyl-1,4-dihydro-6-fluoro-7-(4-morpholinyl)-4-oxo-1,8-naphthyridine-3-carboxylic acid;

1-cyclopropyl-1,4-dihydro-6-fluoro-4-oxo-7-(1-pyrrolidinyl)-1,8-naphthyridine-3-carboxylic acid;

1-cyclopropyl-1,4-dihydro-6-fluoro-4-oxo-7-(1-piperidinyl)-1,8-naphthyridine-3-carboxylic acid; and

1-cyclopropyl-1,4-dihydro-6-fluoro-7-(1-homopiperidinyl)-4-oxo-1,8-naphthyridine-3-carboxylic acid.

PREPARATIVE EXAMPLES FOR INTERMEDIATES

Example A

4-[6-(Cyclopropylamino)-3-nitro-2-pyridinyl]-1-piperazinecarboxylic acid, ethyl ester

A solution of 126.0 g (0.4 mole) of 4-(6-chloro-3-nitro-2-pyridinyl)-1-piperazinecarboxylic acid, ethyl ester (prepared as described in EP—A—0009425), 76.1 g (0.5 mole) of 1,8-diazabicyclo[5.4.0)undec7-ene (DBU), 28.6 g (0.5 mole) of cyclopropylamine and 500 ml of absolute ethanol was stirred at room temperature for 48 hours. The solution was then heated at reflux for four hours and concentrated in vacuo. The residue was partitioned between chloroform and water. The chloroform layer was dried over magnesium sulfate and concentrated in vacuo. The residue was triturated with ether to give 64.0 g of the title compound, mp 100—103°C.

Example B

4-[6-(Acetylcyclopropylamino)-3-nitro-2-pyridinyl]-1-piperazinecarboxylic acid, ethyl ester

A solution of 64.0 g (0.19 mole) of 4-[6-(cyclopropylamino)-3-nitro-2-pyridinyl]-1-piperazine-carboxylic acid, ethyl ester, 115 ml of acetic anhydride and 115 ml of acetic acid was heated on a steam bath for 36 hours. The solvents were removed in vacuo, the residue was triturated with a mixture of ethanol and toluene which was also evaporated in vacuo to give 68.3 g of the title compound, mp 90—93°C.

Example C

4-[6-(Acetylcyclopropylamino)-3-amino-2-pyridinyl]-1-piperazinecarboxylic acid, ethyl ester

A mixture of 17.0 g (45 mmole) of 4-6-(acetylcyclopropylamino)-3-nitro-2-pyridinyl-1-piperazine-carboxylic acid, ethyl ester, 1.5 g of Raney nickel and 180 ml of absolute ethanol was shaken in an atmosphere of hydrogen at about 50 psi and room temperature for approximately 24 hours. The catalyst was removed by filtering through Celite and the solvent removed in vacuo to give 15.2 g of the title compound, mp 149—150°C.

Example D

2-[4-(Ethoxycarbonyl)-1-piperazinyl]-6-(acetylcyclopropylamino)-3-pyridinediazonium tetrafluoroborate

A solution of 20.8 g (60 mmole) of 4-[6-(acetylcyclopropylamino)-3-amino-2-pyridinyl]-1-piperazine-carboxylic acid, ethyl ester, 44 ml of ethanol and 27 ml of 48% tetrafluoroboric acid was cooled to 0°C and treated dropwise with a solution of 4.56 g (66 mmol) of sodium nitrite in 8 ml of water under a nitrogen atmosphere keeping the temperature 0—5°C. After the addition was complete, the reaction was stirred at 0—5°C for one hour and treated with 150 ml of anhydrous ether keeping the temperature below 10°C. The solid was removed by filtration, the precipitate was washed with ethanol/ether (1:1), ether and dried in vacuo to give 24.5 g of the title compound, mp 100—105°C (dec.).

Example E

4-[6-(Acetylcyclopropylamino)-3-fluoro-2-pyridinyl]-1-piperazinecarboxylic acid, ethyl ester

To 800 ml of refluxing toluene was added in portions, as a solid, 46.2 g (0.1 mole) of 2-[4-(ethoxycarbonyl)-1-piperazinyl]-6-(acetylcyclopropylamino)-3-pyridinediazonium tetrafluoroborate. After the addition was complete, the reaction was refluxed for ten minutes and the toluene was decanted from the insoluble precipitate. The toluene was evaporated in vacuo and the residue was partitioned between chloroform and water. The chloroform layer was washed with 5% aqueous sodium bicarbonate, water, dried over magnesium sulfate and evaporated in vacuo to give 13.7 g of the title compound, as a viscous

oil. An additional 10.2 g could be obtained by partitioning the original toluene insoluble material in chloroform and water. The organic layer was washed with 5% aqueous sodium bicarbonate, dried over magnesium sulfate, evaporated in vacuo and the residue was chromatographed on silica gel eluting with chloroform/ethyl acetate (6:4). This fraction was also a viscous oil which did not crystallize upon standing. Both fractions were of sufficient purity to be used as is in the ensuing steps.

Example F

4-[6-(Cyclopropylamino)-3-fluoro-2-pyridinyl]-1-piperazinecarboxylic acid, ethyl ester

A solution of 21.9 g (63 mmole) of 4-[6-(acetylcyclopropylamino)-3-fluoro-2-pyridinyl]-1-piperazinecarboxylic acid, ethyl ester, 170 ml of 15% hydrochloric acid and 235 ml of methanol was refluxed for one hour and stirred at room temperature for 18 hours. The methanol was removed in vacuo and the aqueous acid was made basic with 1.0 *N* sodium hydroxide to pH 10.5. The mixture was extracted with chloroform, the chloroform layer washed with water, dried over magnesium sulfate, and evaporated in vacuo to give 17.6 g of the title compound, mp 68—70°C.

Example G

For Route A

[[Cyclopropyl[6-[4-(ethoxycarbonyl)-1-piperazinyl]-5-fluoro-2-pyridinyl]amino]methylene]propanedioic acid, diethyl ester

A solution of 3.8 g (12.3 mmole) of 4-[6-(cyclopropylamino)-3-fluoro-2-pyridinyl]-1-piperazinecarboxylic acid, ethyl ester, 2.7 g (12.3 mmole) of diethyl (ethoxymethylene)malonate and 50 ml of xylene was refluxed for 24 hours. The solvent was removed in vacuo and the residue was chromatographed over silica gel eluting with chloroform/ethyl acetate (80/20) to give 2.3 g of the title compound as a viscous oil which was used without further purification.

Example H

For Route A

Ethyl 1-Cyclopropyl-6-fluoro-1,4-dihydro-4-oxo-7-[4-(ethoxycarbonyl)-1-piperazinyl]-1,8-naphthyridine-3-carboxylate

A solution of 2.3 g (4.8 mmole) of [[cyclopropyl[6-[4-(ethoxycarbonyl)-1-piperazinyl]-5-fluoro-2-pyridinyl]amino]methylene]propanedioic acid, diethyl ester, in 15 ml of acetic anhydride was treated dropwise with 5 ml of 98% sulfuric acid keeping the temperature at 55—60°C. When the addition was complete, the reaction was stirred for one hour and poured onto 50 g of ice. The aqueous suspension was extracted with chloroform, the chloroform layer washed with water, dried over magnesium sulfate, filtered, and evaporated in vacuo. The residue was triturated with several portions of ethanol/toluene which were also removed in vacuo to give 0.4 g of the title compound, mp 184—186°C. An additional 0.5 g of product could be obtained by concentrating the original aqueous fraction, mp 184—186°C.

Example I

For Route B

4-[6-[Cyclopropyl(2,2-dimethyl-4,6-dioxo-1,3-dioxan-5-ylidine)amino]-3-fluoro-2-pyridinyl]-1-piperazinecarboxylic acid, ethyl ester

A solution of 17.6 g (57 mmole) of 4-[6-(cyclopropylamino)-3-fluoro-2-pyridinyl]-1-piperazinecarboxylic acid, ethyl ester, 11.6 g (63 mmole) of 5-(methoxymethylene)-2,2-dimethyl-1,3-dioxane-4,6-dione and 250 ml of methanol was stirred at room temperature for four hours. The solid was removed by filtration, washed with methanol, ether and dried in vacuo to give 17.6 g of the title compound, mp 177—178°C.

Example J

For Route B

1-Cyclopropyl-6-fluoro-1,4-dihydro-4-oxo-7-[4-(ethoxycarbonyl)-1-piperazinyl]-1,8-naphthyridine-3-carboxylic acid

A solution of 17.0 g (37.0 mmole) of 4-[6-[cyclopropyl (2,2-dimethyl-4,6-dioxo-1,3-dioxan-5-ylidene)-amino]-3-fluoro-2-pyridinyl]-1-piperazinecarboxylic acid, ethyl ester in 125 ml of acetic anhydride was treated dropwise with 35 ml of 98% sulfuric acid keeping the temperature at 50—60°C. When the addition was complete, the reaction was stirred for two hours and poured onto 600 g of ice. The mixture was stirred for one hour and the resulting precipitate was removed by filtration, washed with water and dried in vacuo to give 10.2 g of the title compound, mp 277—279°C.

Example K

1-Cyclopropyl-6-fluoro-1,4-dihydro-7-hydroxy-4-oxo-1,8-naphthyridine-3-carboxylic acid

To a solution of 2 ml of 70% nitric acid in 10 ml of 98% sulfuric acid was added in portions 1.0 g (3.0 mmole) of 1-cyclopropyl-6-fluoro-1,4-dihydro-4-oxo-7-(1-piperazinyl)-1,8-naphthyridine-3-carboxylic acid, keeping the temperature between 25—30°C. The resulting solution was stirred at room temperature for 18 hours and poured onto 40 g of ice. The mixture was stirred at room temperature for 24 hours, concentrated in vacuo, the pH adjusted to 12 with aqueous sodium hydroxide, and filtered through a fiber glass pad. The

filtrate was acidified to pH 3.5 with 6.0 M hydrochloric acid, the resulting precipitate removed by filtration, washed with water then ether and dried in vacuo to give 0.23 g of the title compound, mp 325—327°C.

7-Chloro-1-cyclopropyl-6-fluoro-1,4-dihydro-4-oxo-1,8-naphthyridine-3-carboxylic acid

A suspension of 0.19 g (0.72 mmole) of 1-cyclopropyl-6-fluoro-1,4-dihydro-7-hydroxy-4-oxo-1,8-naphthyridine-3-carboxylic acid in 2 ml of phosphorus oxychloride was heated at reflux for ½ hour. The resulting solution was cooled to room temperature and the solvent was removed in vacuo. The residue was triturated with ice-water and the resulting solid was removed by filtration, washed with water, then ether and dried in vacuo to give 0.11 g of the title compound, mp 209—212°C.

Example L

1-Cyclopropyl-6,7,8-trifluoro-1,4-dihydro-4-oxo-3-quinolinecarboxylic Acid

2,3,4,5-Tetrafluoro-β-oxo-benzenepropanoic Acid, Ethyl Ester

To 30.0 g (155 mmol) of 2,3,4,5-tetrafluorobenzoic acid in 75 ml of dichloromethane was added 14.8 ml (1.1 equivalents) of oxalyl chloride. The mixture was then treated with three drops of dry N,N-dimethylformamide and the vigorous reaction was stirred at room temperature overnight. The mixture was then concentrated to an oil, taken up in toluene, and reconcentrated to afford 2,3,4,5-tetrafluorobenzoyl chloride which was used in the next step.

To 40.92 g (310 mmol) of malonic acid half ethyl ester in 700 ml of dry tetrahydrofuran at −35°C was added a stream of n-butyllithium until one equivalent was delivered. The mixture was maintained at −15 to −30°C during the addition, then warmed to −5°C and treated with 10 mg of bipyridyl. The remainder of the n-butyllithium was added at this temperature until the indicator turned pink. A total of 282 ml of 2.2 N n-butyllithium was added. The mixture was recooled to −78°C and a solution of 2,3,4,5-tetrafluorobenzoyl chloride in 100 ml of dry tetrahydrofuran was added keeping the temperature constant. The reaction mixture was stirred for 45 minutes after the acid chloride addition. It was warmed to −35°C and poured into 155 ml of 2 N hydrochloric acid. To this mixture was added one liter of water and 1.5 liters of dichloromethane. The aqueous phase was separated and extracted with additional 1.5 liters of dichloromethane. The combined organic phases were washed with 50% saturated sodium bicarbonate and then 1 N hydrochloric acid. The dichloromethane was dried (magnesium sulfate) and concentrated to a solid which was triturated with cold pentane to give 37.8 g of 2,3,4,5-tetrafluoro-β-oxo-benzenepropanoic acid, ethyl ester, mp 63—65°C.

1-Cyclopropyl-6,7,8-trifluoro-1,4-dihydro-4-oxo-3-quinolinecarboxylic Acid

To 17.6 g (66.6 mmol) of the 2,3,4,5-tetrafluoro-β-oxo-benzenepropanoic acid was added 14.6 g (~1.5 equivalents) of triethylorthoformate and 16.19 g (2.38 equivalents) of acetic anhydride. The mixture was refluxed for two hours at 120°C (and was then cooled to 80°C and concentrated in vacuo. The mixture was diluted with t-butanol, cooled to 10°C, and 3.8 g (1.05 equivalents) of cyclopropylamine in 120 ml of t-butanol was added. The mixture was stirred at 20°C for 30 minutes and then warmed to 50°C overnight. At this temperature 7.5 g of potassium t-butoxide was added in 50 ml of t-butanol and the mixture was stirred for four hours. It was filtered and the solids dissolved in 250 ml of hot acetic acid and 200 ml of 3 N hydrochloric acid were added in portions over four hours at 100°C. The mixture was cooled and the solids collected to give 15.44 g (82%) of the 1-cyclopropyl-6,7,8-trifluoro-1,4-dihydro-4-oxo-3-quinolinecarboxylic acid, mp 226—228°C.

**Claims for the Contracting States: BE CH DE FR GB IT LI LU NL SE**

1. A compound of the formula I

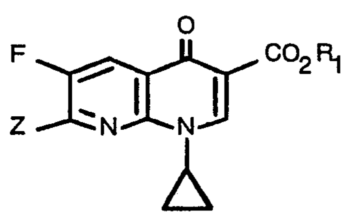

(I)

wherein Z is a leaving group or a group of the formula

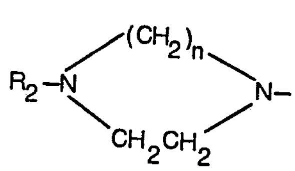

where n is 2—3 and $R^2$ is hydrogen, $C_1$—$C_3$ alkyl or acetyl,

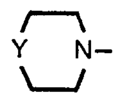

where Y is O or S, or

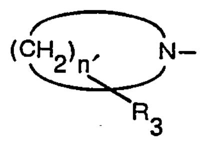

where n' is 4—6 and $R^3$ is hydrogen or hydroxyl; $R^1$ is hydrogen or $C_1$—$C_3$ alkyl, or a pharmaceutically acceptable acid-addition or base salt thereof other than the pharmaceutically acceptable salts of the compounds of formula I in which Z is a leaving group.

2. A compound of the formula I

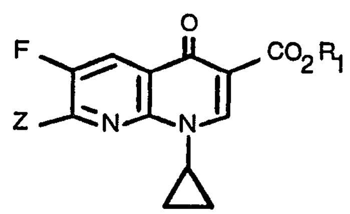

(I)

wherein Z is a leaving group or a group of the formula

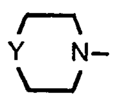

where Y is O or S, or

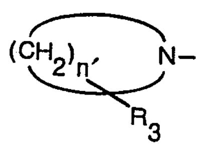

where n' is 4—6 and $R^3$ is hydrogen or hydroxyl; $R_1$ is hydrogen or $C_1$—$C_3$ alkyl, or a pharmaceutically acceptable acid-addition or base salt thereof other than the pharmaceutically acceptable salts of the compounds of formula I in which Z is a leaving group.

3. A pharmaceutical composition comprising a compound as claimed in Claim 1 or 2, other than one in which Z is a leaving group, together with a pharmaceutically acceptable carrier or diluent.

4. A process for the preparation of a compound as claimed in claim 1 which comprises reacting a compound of the formula II

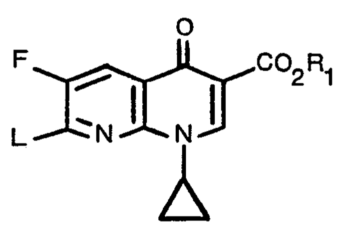

(II)

wherein L is a leaving group, preferably fluorine or chlorine with an amine, or blocked amine, corresponding to group Z, and, if desired, converting the resulting product to a pharmaceutically acceptable acid addition or base salt thereof.

5. A compound according to Claim 1 having one of the following names:

1-cyclopropyl-1,4-dihydro-6-fluoro-4-oxo-7-(1-piperazinyl)-1,8-naphthyridine-3-carboxylic acid;

1-cyclopropyl-1,4-dihydro-6-fluoro-4-oxo-7-(4-methyl-1-piperazinyl)-1,8-naphthyridine-3-carboxylic acid;

1-cyclopropyl-1,4-dihydro-6-fluoro-4-oxo-7-(4-thiomorpholinyl)-1,8-naphthyridine-3-carboxylic acid;
1-cyclopropyl-1,4-dihydro-6-fluoro-4-oxo-7-(4-morpholinyl)-1,8-naphthyridine-3-carboxylic acid;
1-cyclopropyl-1,4-dihydro-6-fluoro-4-oxo-7-(1-pyrrolidinyl)-1,8-naphthyridine-3-carboxylic acid;
1-cyclopropyl-1,4-dihydro-6-fluoro-4-oxo-7-(1-piperidinyl)-1,8-naphthyridine-3-carboxylic acid; and
1-cyclopropyl-1,4-dihydro-6-fluoro-4-oxo-7-(1-homopiperidinyl)-1,8-naphthyridine-3-carboxylic acid.

6. A compound of Formula I as claimed in Claim 1, other than those compounds in which Z is a leaving group for use as an antibacterial agent.

7. A process for preparing a compound of the formula II

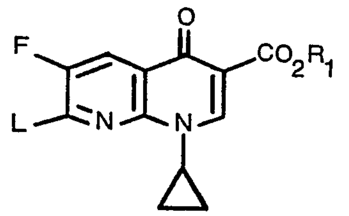

(II)

wherein L is fluorine or chlorine and $R^1$ is a hydrogen or $C_1$—$C_3$ alkyl; which process comprises displacing the hydroxyl group at the 7-position of a compound of the formula

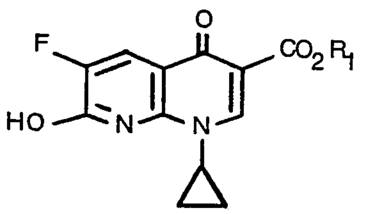

by a fluorine or chlorine.

8. A process according to claim 7, wherein L is chlorine and the hydroxyl group is displaced by treatment of the compound of formula I with phosphorus oxychloride.

**Claims for the Contracting State: AT**

1. A process for the preparation of a compound of the formula I

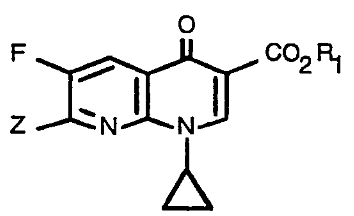

(I)

wherein Z is a group of the formula

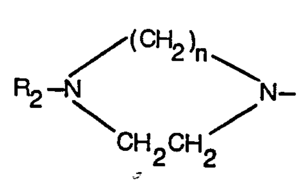

where n is 2—3 and $R^2$ is hydrogen, $C_1$—$C_3$ alkyl or acetyl,

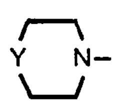

where Y is O or S, or

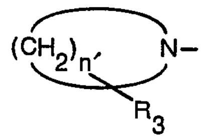

where n′ is 4—6 and $R^3$ is hydrogen or hydroxyl; $R^1$ is hydrogen or $C_1$—$C_3$ alkyl, or a pharmaceutically acceptable acid-addition or base salt thereof; which process comprises reacting a compound of the formula II

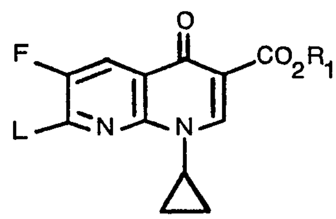

(II)

wherein L is a leaving group, preferably fluorine or chlorine with an amine or blocked amine corresponding to group Z, and, if desired, converting the resulting product to a pharmaceutically acceptable acid addition or base salt thereof.

2. A process for the preparation of a compound of the formula I

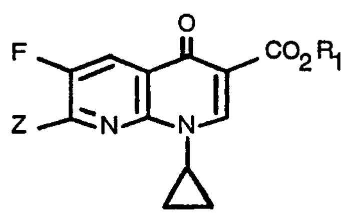

(I)

wherein Z is a group of the formula

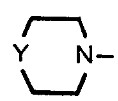

where Y is O or S, or

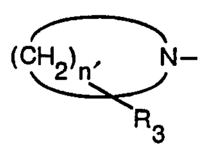

where n′ is 4—6 and $R^3$ is hydrogen or hydroxyl; $R_1$ is hydrogen or $C_1$—$C_3$ alkyl, or a pharmaceutically acceptable acid-addition or base salt thereof; which process comprises reacting a compound of the formula II

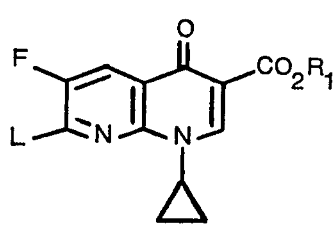

(II)

wherein L is a leaving group, preferably fluorine or chlorine with an amine or blocked amine corresponding to group Z, and, if desired, converting the resulting product to a pharmaceutically acceptable acid addition or base salt thereof.

3. A process for preparing a pharmaceutical composition which comprises combining a compound prepared by a process as claimed in any preceding claim together with a pharmaceutically acceptable carrier or diluent.

4. A process according to Claim 1 for preparing a compound having one of the following names:
1-cyclopropyl-1,4-dihydro-6-fluoro-4-oxo-7-(1-piperazinyl)-1,8-naphthyridine-3-carboxylic acid;
1-cyclopropyl-1,4-dihydro-6-fluoro-4-oxo-7-(4-methyl-1-piperazinyl)-1,8-naphthyridine-3-carboxylic acid;
1-cyclopropyl-1,4-dihydro-6-fluoro-4-oxo-7-(4-thiomorpholinyl)-1,8-naphthyridine-3-carboxylic acid;
1-cyclopropyl-1,4-dihydro-6-fluoro-4-oxo-7-(4-morpholinyl)-1,8-naphthyridine-3-carboxylic acid;
1-cyclopropyl-1,4-dihydro-6-fluoro-4-oxo-7-(1-pyrrolidinyl)-1,8-naphthyridine-3-carboxylic acid;
1-cyclopropyl-1,4-dihydro-6-fluoro-4-oxo-7-(1-piperidinyl)-1,8-naphthyridine-3-carboxylic acid; and
1-cyclopropyl-1,4-dihydro-6-fluoro-4-oxo-7-(1-homopiperidinyl)-1,8-naphthyridine-3-carboxylic acid.

5. A process for preparing a compound of the formula II

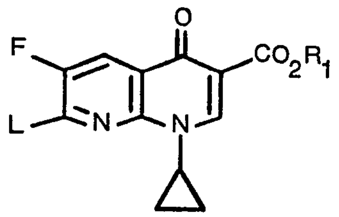

(II)

wherein L is fluorine or chlorine and $R^1$ is a hydrogen or $C_1$—$C_3$ alkyl; which process comprises displacing the hydroxyl group at the 7-position of a compound of the formula

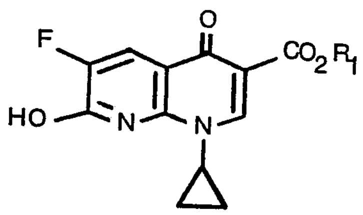

by a fluorine or chlorine.

6. A process according to claim 5, wherein L is chlorine and the hydroxyl group is displaced by treatment of the compound of formula I with phosphorus oxychloride.

**Patentansprüche für die Vertragsstaaten: BE CH DE FR GB IT LI LU NL SE**

1. Verbindung der Formel I

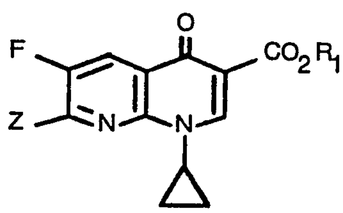

(I)

worin Z ist: eine austretende Gruppe oder eine Gruppe der Formel

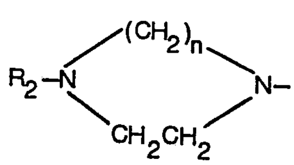

worin n für 2—3 steht, und $R^2$ ist: Wasserstoff, $C_1$—$C_3$ Alkyl oder Acetyl,

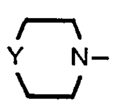

worin Y für O oder S steht, oder

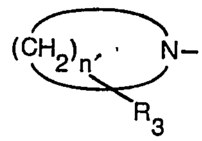

worin n' für 4—6 steht, und $R^3$ Wasserstoff oder Hydroxyl ist;

$R^1$ ist: Wasserstoff oder $C_1$–$C_3$ Alkyl, oder ein pharmazeutisch akzeptables Säureadditions- oder basisches Salz davon, außer den pharmazeutisch akzeptablen Salzen der Verbindungen der Formel I, worin Z eine austretende Gruppe ist.

2. Verbindung der Formel I

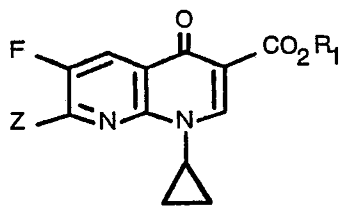

(I)

worin Z ist: eine austretende Gruppe oder eine Gruppe der Formel

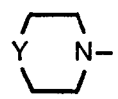 ,

worin Y O oder S ist, oder

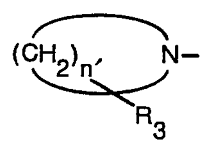 ,

worin n' für 4—6 steht, und $R^3$ Wasserstoff oder Hydroxyl ist;

$R_1$ für Wasserstoff oder $C_1$–$C_3$ Alkyl steht, oder ein pharmazeutisch akzeptables Säureadditions- oder basisches Salz davon, außer den pharmazeutisch akzeptablen Salzen der Verbindungen der Formel I, worin Z eine austretende Gruppe ist.

3. Pharmazeutische Zusammensetzung, welche eine wie im Anspruch 1 oder 2 beanspruchte Verbindung, welche nicht jene ist, in der Z eine austretende Gruppe ist, zusammen mit einem pharmazeutisch akzeptablen Träger oder Verdünnungsmittel umfaßt.

4. Verfahren zur Herstellung einer wie im Anspruch 1 beanspruchten Verbindung, welches die Umsetzung einer Verbindung der Formel II

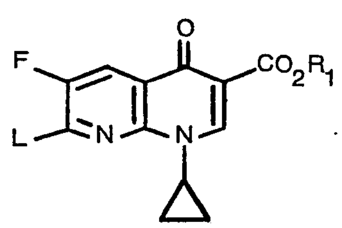

(II)

worin L eine austretende Gruppe, vorzugsweise Fluor oder Chlor ist, mit einem Amin oder blockierten Amin, welches der Gruppe Z entspricht, und gewünschtenfalls das Überführen des resultierenden Produkts in ein pharmazeutisch akzeptables Säureadditions- oder basisches Salz davon umfaßt.

5. Verbindung nach Anspruch 1 mit einem der folgenden Namen:

1-Cyclopropyl-1,4-dihydro-6-fluor-4-oxo-7-(1-piperazinyl)-1,8-naphthyridine-3-carbonsäure;

1-Cyclopropyl-1,4-dihydro-6-fluor-4-oxo-7-(4-methyl-1-piperazinyl)-1,8-naphthyridine-3-carbonsäure;

1-Cyclopropyl-1,4-dihydro-6-fluor-4-oxo-7-(4-thiomorpholinyl)-1,8-naphthyridine-3-carbonsäure;

1-Cyclopropyl-1,4-dihydro-6-fluor-4-oxo-7-(4-morpholinyl)-1,8-naphthyridine-3-carbonsäure;

1-Cyclopropyl-1,4-dihydro-6-fluor-4-oxo-7-(1-pyrrolidinyl)-1,8-naphthyridine-3-carbonsäure;

1-Cyclopropyl-1,4-dihydro-6-fluor-4-oxo-7-(1-piperidinyl)-1,8-naphthyridine-3-carbonsäure; und
1-Cyclopropyl-1,4-dihydro-6-fluor-4-oxo-7-(1-homopiperidinyl)-1,8-naphthyridine-3-carbonsäure.

6. Verbindung der wie im Anspruch 1 beanspruchen Formel I, welche sich von jenen Verbindungen, worin Z eine austretende Gruppe ist, unterscheidet, zur Verwendung als antibakterielles Mittel.

7. Verfahren zur Herstellung einer Verbindung der Formel II

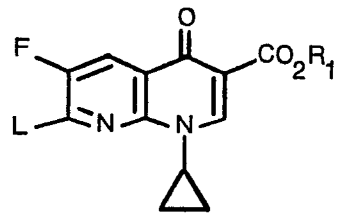

(II)

worin L für Fluor oder Chlor steht, und $R^1$ ein Wasserstoff oder $C_1$—$C_3$ Alkyl ist; welches Verfahren das Ersetzen der Hydroxylgruppe an der Position 7 einer Verbindung der Formel

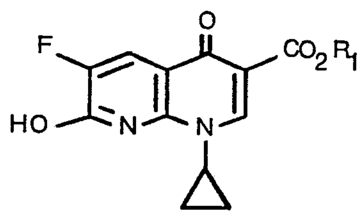

mit einem Fluor oder Chlor umfaßt.

8. Verfahren nach Anspruch 7, worin L für Chlor steht, und die Hydroxylgruppe durch Behandlung der Verbindung der Formel I mit Phosphoroxychlorid ersetzt wird.

**Patentansprüche für den Vertragsstaat: AT**

1. Verfahren zur Herstellung einer Verbindung der Formel I

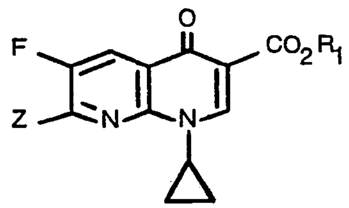

(I)

worin Z ist: eine Gruppe der Formel

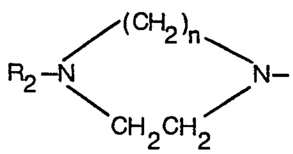

worin n für 2—3 steht, und $R^2$ ist: Wasserstoff, $C_1$—$C_3$ Alkyl oder Acetyl,

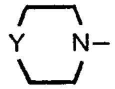

worin Y für O oder S steht, oder

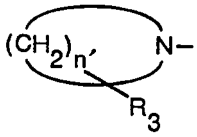

worin n' für 4—6 steht, und $R^3$ Wasserstoff oder Hydroxyl ist;

$R^1$ ist: Wasserstoff oder $C_1$–$C_3$ Alkyl, oder eines pharmazeutisch akzeptables Säureadditions- oder basischens Salzes davon, welches Verfahren das Umsetzen einer Verbindung der Formel II

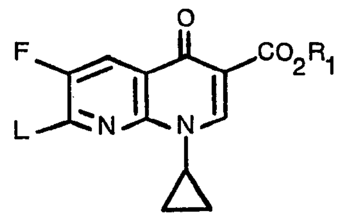

(II)

worin L eine austretende Gruppe, vorzugsweise Fluor oder Chlor ist, mit einem Amin oder blockierten Amin, welches der Gruppe Z entspricht, und gewünschtenfalls das Überführen des resultierenden Produkts in ein pharmazeutisch akzeptables Säureadditions- oder basisches Salz davon umfaßt.

2. Verfahren zur Herstellung einer Verbindung der Formel I

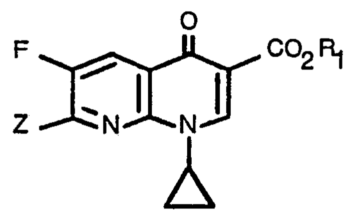

(I)

worin Z eine Gruppe der Formel

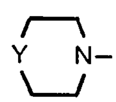

,

worin Y O oder S ist, oder

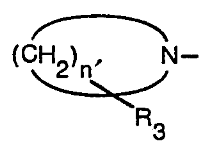

,

worin n' für 4—6 ist, und $R^3$ Wasserstoff oder Hydroxyl ist;

$R_1$ für Wasserstoff oder $C_1$—$C_3$ Alkyl ist, oder eines pharmazeutisch akzeptablen Säureadditions- oder basischen Salzes davon; welches Verfahren die Umsetzung einer Verbindung der Formel II

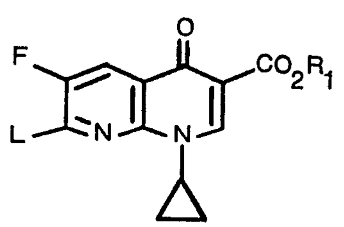

(II)

worin L eine austretende Gruppe, vorzugsweise Fluor oder Chlor ist, mit einem Amin oder blockierten Amin, welches der Gruppe Z entspricht, und gewünschtenfalls das Überführen des resultierenden Produkts in ein pharmazeutisch akzeptables Säureadditions- oder basisches Salz davon umfaßt.

3. Verfahren zur Herstellung einer pharmazeutischen Zusammensetzung, welches Verfahren das Vereinigen einer mittels eines in irgendeinem der vorhergehenden Ansprüche beanspruchten Verfahrens hergestellten Verbindung mit einem pharmazeutisch akzeptablen Träger oder Verdünnungsmittel umfaßt.

4. Verfahren nach Anspruch 1 zur Herstellung einer Verbindung mit einem der folgenden Namen:

1-Cyclopropyl-1,4-dihydro-6-fluor-4-oxo-7-(1-piperazinyl)-1,8-naphthyridine-3-carbonsäure;

1-Cyclopropyl-1,4-dihydro-6-fluor-4-oxo-7-(4-methyl-1-piperazinyl)-1,8-naphthyridine-3-carbonsäure;

1-Cyclopropyl-1,4-dihydro-6-fluor-4-oxo-7-(4-thiomorpholinyl)-1,8-naphthyridine-3-carbonsäure;

1-Cyclopropyl-1,4-dihydro-6-fluor-4-oxo-7-(4-morpholinyl)-1,8-naphthyridine-3-carbonsäure;

1-Cyclopropyl-1,4-dihydro-6-fluor-4-oxo-7-(1-pyrrolidinyl)-1,8-naphthyridine-3-carbonsäure;

1-Cyclopropyl-1,4-dihydro-6-fluor-4-oxo-7-(1-piperidinyl)-1,8-naphthyridine-3-carbonsäure; und
1-Cyclopropyl-1,4-dihydro-6-fluor-4-oxo-7-(1-homopiperidinyl)-1,8-naphthyridine-3-carbonsäure.

5. Verfahren zur Herstellung einer Verbindung der Formel II

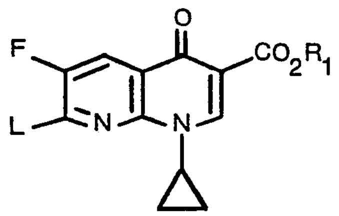

(II)

worin L für Fluor oder Chlor steht, und $R^1$ ein Wasserstoff oder $C_1$—$C_3$ Alkyl ist; welches Verfahren das Ersetzen der Hydroxylgruppe an der Position 7 einer Verbindung der Formel

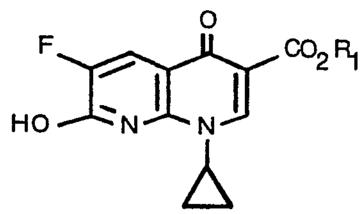

durch ein Fluor oder Chlor umfaßt.

6. Verfahren nach Anspruch 5, worin L Chlor ist, und die Hydroxylgruppe durch Behandlung der Verbindung der Formel I mit Phosphoroxychlorid ersetzt wird.

**Revendications pour les Etats contractants: BE CH DE FR GB IT LI LU NL**

1. Un dérivé de formule I:

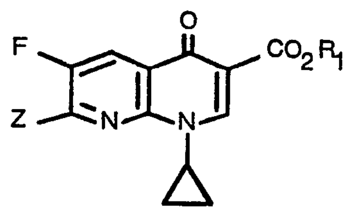

(I)

dans laquelle:

Z est un groupe partant ou un groupement de formule:

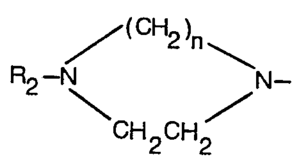

dans laquelle:

n vaut 2 ou 3; et

$R_2$ est un atome d'hydrogène, un groupement alkyle en $C_1$ à $C_3$ ou un groupement acétyle, ou

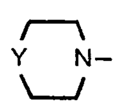

dans lequel Y est O ou S, ou

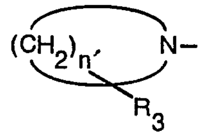

dans lequel n' vaut de 4 à 6, et $R_3$ est un atome d'hydrogène ou un hydroxyle;

$R^1$ est un atome d'hydrogène ou un groupement alkyle en $C_1$ à $C_3$, ou un de ses sels d'addition d'acide ou de base pharmaceutiquement acceptables, autre que les sels pharmaceutiquement acceptables des dérivés correspondant à la formule I dans laquelle Z est un groupe partant.

2. Un dérivé de formule I:

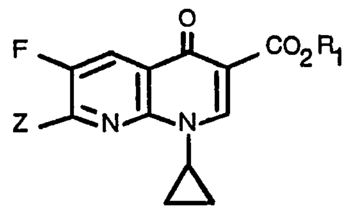

(I)

dans laquelle:

Z est un groupe partant ou un groupe de formule:

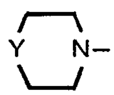

dans lequel Y est O ou S, ou

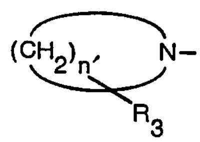

dans lequel n' vaut de 4 à 6, et $R_3$ est un atome d'hydrogène ou un hydroxyle;

$R^1$ est un atome d'hydrogène ou un groupement alkyle en $C_1$ à $C_3$, ou un de ses sels d'addition d'acide ou de base pharmaceutiquement acceptables, autre que les sels pharmaceutiquement acceptables des dérivés correspondant à la formule I dans laquelle Z est un groupe partant.

3. Une composition pharmaceutique incluant un dérivé selon la revendication 1 ou 2, autre qu'un dérivé dans lequel Z est un groupe partant, mélangé avec un support ou un diluant pharmaceutiquement acceptable.

4. Un procédé de préparation d'un dérivé selon la revendication 1, caractérisé en ce que l'on fait réagir un dérivé de formule II:

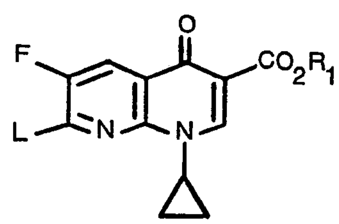

(II)

dans laquelle:

L est un groupe partant, de préférence un fluor ou un chlore, avec une amine ou une amine bloquée correspondant au groupe Z, et, si on le souhaite, on transforme le produit obtenu en un de ses sels d'addition d'acide ou de base pharmaceutiquement acceptables.

5. Un dérivé selon à la revendication 1 et ayant l'un des noms suivants:

acide 1-cyclopropyl-1,4-dihydro-6-fluoro-4-oxo-7-(1-pipérazinyl)-1,8-naphtyridine-3-carboxylique;

acide 1-cyclopropyl-1,4-dihydro-6-fluoro-4-oxo-7-(4-méthyl-1-pipérazinyl)-1,8-naphtyridine-3-carboxylique;

acide 1-cyclopropyl-1,4-dihydro-6-fluoro-4-oxo-7-(4-thiomorpholinyl)-1,8-naphtyridine-3-carboxylique;

acide 1-cyclopropyl-1,4-dihydro-6-fluoro-4-oxo-7-(4-morpholinyl)-1,8-naphtyridine-3-carboxylique;

acide 1-cyclopropyl-1,4-dihydro-6-fluoro-4-oxo-7-(1-pyrrolidinyl)-1,8-naphtyridine-3-carboxylique;
acide 1-cyclopropyl-1,4-dihydro-6-fluoro-4-oxo-7-(1-pipéridinyl)-1,8-naphtyridine-3-carboxylique;
acide 1-cyclopropyl-1,4-dihydro-6-fluoro-4-oxo-7-(1-homopipéridinyl)-1,8-naphtyridine-3-carboxylique;

6. Un dérivé de formule I selon à la revendication 1, autre que ceux des dérivés dans lesquels Z est un groupe partant, pour un usage en tant qu'agent antibactérien.

7. Un procédé pour la préparation d'un dérivé de formule II:

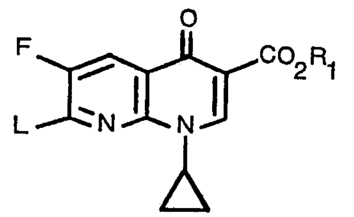

(II)

dans laquelle:
L est un fluor ou un chlore; et
$R_1$ est un atome d'hydrogène ou un groupe alkyle en $C_1$ à $C_3$;
procédé dans lequel on remplace le groupe hydroxyle en position 7 d'un dérivé de formule

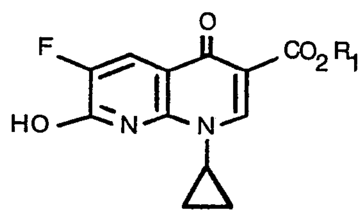

par un fluor ou un chlore.

8. Un procédé selon la revendication 7, caractérisé en ce que L est un chlore et dans lequel le groupe hydroxyle est remplacé par le traitement du dérivé de formule I par l'oxychlorure de phosphore.

**Revendications pour l'Etat contractant: AT**

1. Un procédé de préparation d'un dérive de formule I:

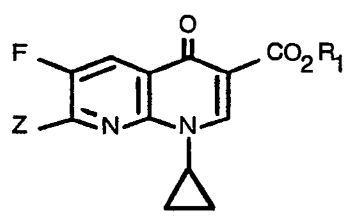

(I)

dans laquelle:
Z est un groupe partant ou un groupement de formule:

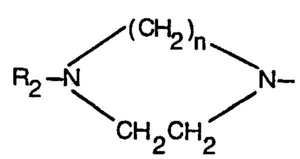

dans laquelle:
n vaut 2 ou 3; et
$R_2$ est un atome d'hydrogène, un groupement alkyle en $C_1$ à $C_3$ ou un groupement acétyle, ou

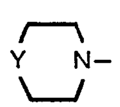

dans lequel Y est O ou S, ou

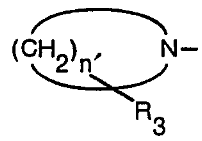

dans lequel n' vaut de 4 à 6, et $R_3$ est un atome d'hydrogène ou un hydroxyle;

$R^1$ est un atome d'hydrogène ou un groupement alkyle en $C_1$ à $C_3$, ou un de ses sels d'addition d'acide ou de base pharmaceutiquement acceptables, lequel procédé comprend la réaction avec un dérive de formule II:

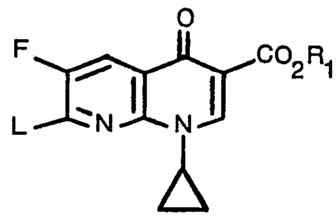

(II)

dans laquelle:

L est un groupe partant, de préférence un fluor ou un chlore, avec une amine ou une amine bloquée correspondant au groupe Z, et, si on le souhaie, on transforme le produit obtenu en un de ses sels d'addition d'acide ou de base pharmaceutiquement acceptables.

2. Un procédé de préparation d'un dérive de formule I:

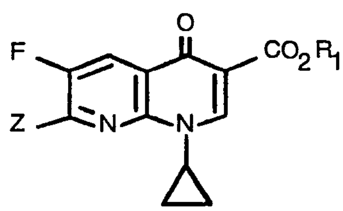

(I)

dans laquelle:

Z est un groupe partant ou un groupe de formule:

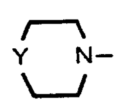

dans lequel Y est O ou S, ou

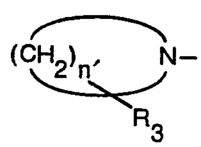

dans lequel n' vaut de 4 à 6, et $R_3$ est un atome d'hydrogène ou un hydroxyle;

$R^1$ est un atome d'hydrogène ou un groupement alkyle en $C_1$ à $C_3$, lequel procédé comprend la réaction avec un dérivé de formule II:

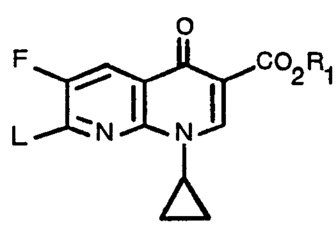

(II)

dans laquelle:

L est un groupe partant, de préférence un fluor ou un chlore, avec une amine ou une amine bloquée correspondant au groupe Z, et, si on le souhaite, on transforme le produit obtenu en un de ses sels d'addition d'acide ou de base pharmaceutiquement acceptables.

3. Un procédé de préparation d'une composition pharmaceutique qui consiste à associer un dérivé préparé par un procédé selon l'une quelconque des revendications précédentes, avec un support ou un diluant pharmaceutiquement acceptable.

4. Un procédé selon la revendication 1, pour la préparation d'un dérivé ayant l'un des noms suivants:

acide 1-cyclopropyl-1,4-dihydro-6-fluoro-4-oxo-7-(1-pipérazinyl)-1,8-naphtyridine-3-carboxylique;

acide 1-cyclopropyl-1,4-dihydro-6-fluoro-4-oxo-7-(4-méthyl-1-pipérazinyl)-1,8-naphtyridine-3-carboxylique;

acide 1-cyclopropyl-1,4-dihydro-6-fluoro-4-oxo-7-(4-thiomorpholinyl)-1,8-naphtyridine-3-carboxylique;

acide 1-cyclopropyl-1,4-dihydro-6-fluoro-4-oxo-7-(4-morpholinyl)-1,8-naphtyridine-3-carboxylique;

acide 1-cyclopropyl-1,4-dihydro-6-fluoro-4-oxo-7-(1-pyrrolidinyl)-1,8-naphtyridine-3-carboxylique;

acide 1-cyclopropyl-1,4-dihydro-6-fluoro-4-oxo-7-(1-pipéridinyl)-1,8-naphtyridine-3-carboxylique;

acide 1-cyclopropyl-1,4-dihydro-6-fluoro-4-oxo-7-(1-homopipéridinyl)-1,8-naphtyridine-3-carboxylique;

5. Un procédé de préparation d'un dérivé de formule II:

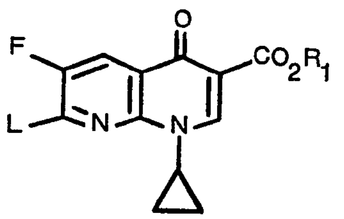

(II)

dans laquelle:

L est un fluor ou un chlore; et

$R_1$ est un atome d'hydrogène ou un groupe alkyle en $C_1$ à $C_3$;

procédé dans lequel on remplace le groupe hydroxyle en position 7 d'un dérivé de formule:

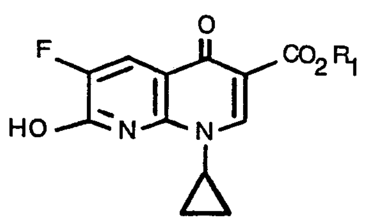

par un fluor ou un chlore.

6. Un procédé selon la revendication 5, caractérisé en ce que L est un chlore et dans lequel le groupe hydroxyle est remplacé par le traitement du dérivé de formule I par l'oxychlorure de phosphore.